# EUROPÄISCHE PATENTSCHRIFT

(11) **EP 2 961 344 B1**
(45) Veröffentlichungstag und Bekanntmachung des Hinweises auf die Patenterteilung: **04.10.2017**
(21) Anmeldenummer: 14711174.4
(22) Anmeldetag: 03.03.2014
(51) Int. Cl.: A61C 13/00

(54) **VERFAHREN ZUR KONSTRUKTION VON ZAHNOBERFLÄCHEN EINES ZAHNERSATZTEILS SOWIE ZUR HERSTELLUNG DENTALER RESTAURATIONEN**
METHOD FOR CONSTRUCTING TOOTH SURFACES OF A DENTAL PROSTHESIS AND FOR PRODUCING DENTAL RESTORATIONS
PROCÉDÉ PERMETTANT LA CONSTRUCTION DE FACES DENTAIRES D'UN ÉLÉMENT PROTHÉTIQUE DENTAIRE ET LA RÉALISATION DE RESTAURATIONS DENTAIRES

(30) Priorität: 01.03.2013 DE 102013203588
(43) Veröffentlichungstag der Anmeldung: 06.01.2016
(73) Patentinhaber: Sirona Dental Systems GmbH, 64625 Bensheim (DE)
(72) Erfinder: HAUTH, Steffen, 55124 Mainz (DE); SCHNEIDER, Sascha, 64367 Mühltal (DE)
(74) Vertreter: Sommer, Peter
(86) Internationale Anmeldenummer: PCT/EP2014/054033
(87) Internationale Veröffentlichungsnummer: WO 2014/131908

(56) Entgegenhaltungen:
- EP-A1- 1 700 576
- EP-A1- 2 389 892
- WO-A1-02/102270

## Beschreibung

### Technisches Gebiet

Die vorliegende Erfindung betrifft ein Verfahren zur Konstruktion von Zahnoberflächen eines Zahnersatzteils sowie zur Herstellung dentaler Restaurationen, ausgehend von einem 3D-Datensatz einer Oberkiefersituation und einem 3D-Datensatz einer Unterkiefersituation jeweils mit mehreren im jeweiligen Kieferbogen angeordneten Zähnen, wobei die Zähne der Oberkiefersituation und die Zähne der Unterkiefersituation bei Interkuspidation einen Vielpunktkontakt mit vielen Kontaktpunkten aufweisen. Weiterhin betrifft die Erfindung ein Computerprogramm sowie einen Datenträger und die Verwendung eines Rechners Steuerung des Computerprogramms. Die ausreichende Zuordnung der Kiefersituationen zueinander ist jedoch erforderlich, um Zahnflächen von Zahnersatzteilen zu konstruieren und herzustellen oder Zahnstellungen zu korrigieren.

### Stand der Technik

Im Zusammenhang mit dem Stand der Technik werden die EP1700576 A1 und die EP2389892 A1 genannt. Bisherige Werkzeuge, die dazu bekannt sind, unterscheiden sich in Komfort oder Präzision, ziehen die sich gegenüberliegenden Kiefer ohne Berücksichtigung der Morphologie strikt bis zu ihrer rigiden Berührung in Kontakt oder müssen komplett manuell gesteuert werden.

Bei der bukkalen Registrierung und bei einem Bissregistrat, auch als Quetschbiss bezeichnet, wird mittels einer oder mehreren bukkalen Aufnahmen oder Bissregistraten eine Ausrichtung der beiden Kiefer zueinander errechnet. Diese Art des zueinander in Beziehung Setzens hat allerdings numerische Grenzen. Die lokale Auflösungsgenauigkeit an der Stelle der Aufnahme bestimmt zwangsläufig über die Hebelwirkung die erreichbare Genauigkeit der Passung an der beabstandeten gegenüberliegenden Stelle. Geht man beispielsweise von einer lokalen Genauigkeit von 25µm aus, so erreicht man auf der gegenüberliegenden Kieferseite, die im Durchschnitt 8cm entfernt liegt, eine Auswirkung von 200µm. Eine Verdopplung der Scangenauigkeit auf 12µm erzeugt auf der gegenüberliegenden Seite immer noch 100µm Auswirkung. Eine mögliche Optimierung ist die Verwendung mehrerer Stellen zur bukkalen Registrierung oder für das Bissregistrat, um den Fehler auszumitteln. Dieses Verfahren ist nur anwendbar, wenn bukkale Registrierungen oder Quetschbiss-Informationen vorliegen.

Eine weitere Möglichkeit das Problem zu lösen ist es, direkt oder nach einer Vorregistrierung die freie Bewegbarkeit der Kiefer zueinander in 3 Raumachsen translierebnd, also längsverschieblich und um 3 Raumachsen rotatorisch zu erlauben. Der Nutzer muss rein visuell frei per Hand schwebend im virtuellen 3D Raum die Kontaktsitutation herstellen, ohne dabei auf ein physikalisches Kontaktgefühl, das er an einem echten Modell hatte, vertrauen zu können.

Aus der DE 10 2005 011 066 A1 ist es bekannt, drei gewünschte Punkte manuell auf einem ersten und zweiten 3D-Datensatz eines Modells festzulegen, wobei die Punkte sowohl in dem ersten als auch in dem zweiten 3D-Datensatz enthalten sind und die Korrelation der 3D-Datensätze herzustellen. Obwohl in einem Modell eine Zahnfläche des Gegenkiefers enthalten sein kann, werden keine unterschiedlichen Modelle zugeordnet.

Nachteilig ist, dass in keinem der bisher bekannten Fälle die angestrebte maximale Interkuspidation beachtet werden kann, wie es im normalen Laborbetrieb mit in einem Artikulator eingespannten Gipsmodellen geschieht. Daher bleibt die Festlegung der gewünschten Kontaktstellen hinter dem manuellen Einpassen zurück und oder erfüllt die methodische Zielsetzung nur mangelhaft.

Es ist die Aufgabe der vorliegenden Erfindung, ein Werkzeug bereitzustellen, mit dem ein Nutzer für eine gegebene Unter- und Oberkiefersituation eine gewünschten Kontaktsituation erzeugen und damit die Zuordnung zueinander festlegen kann, um Zahnflächen von Zahnersatzteilen zu konstruieren und herzustellen oder Zahnstellungen zu korrigieren.

### Darstellung der Erfindung

Diese Aufgabe wird durch das erfindungsgemäße Verfahren zur Konstruktion von Zahnoberflächen eines Zahnersatzteils sowie zur Herstellung dentaler Restaurationen gelöst.

Ausgehend von einem 3D-Datensatz einer Oberkiefersituation und einem 3D-Datensatz einer Unterkiefersituation jeweils mit mehreren im jeweiligen Kieferbogen angeordneten Zähnen, wobei die Zähne der Oberkiefersituation und die Zähne der Unterkiefersituation bei Interkuspidation einen Vielpunktkontakt mit vielen Kontaktpunkten aufweisen, erfolgt die Zuordnung mit einer Kontaktoptimierung, indem
- aus dem 3D-Datensatz der Oberkiefersituation und aus dem 3D-Datensatz der Unterkiefersituation eine gemeinsame Okklusalrichtung automatisch bestimmt wird,
- die Position der Zähne im Kieferbogen im 3D-Datensatz der Oberkiefersituation und im 3D-Datensatz der Unterkiefersituation automatisch bestimmt wird,
- anhand der Position der Zähne im Kieferbogen sich erwartungsgemäß zumindest näherungsweise gegenüberliegende Flächenelemente auf mindestens einer Seite automatisch identifiziert werden und
- auf mindestens einer Seite der gegenüberliegenden Flächenelemente diejenigen Flächenelemente, die in Kontakt treten sollen, vom Nutzer und/oder automatisch näherungsweise ausgewählt werden;
- auf der anderen Seite der gegenüberliegenden Flächenelemente diejenigen Flächenelemente, die in Kontakt treten sollen, vom Nutzer und/oder automatisch näherungsweise ausgewählt werden;
- Bereiche, die auf beiden Seiten der gegenüberliegenden Flächenelemente vorhanden sind, ausgeschnitten werden und Flächenpaare bilden,
- nur die ausgeschnittenen Flächenpaare mittels eines Optimierungsalgorithmus im Abstand zueinander minimiert werden, dass
- die optimierte Zuordnung der Flächenpaare auf den gesamten 3D-Datensatz der Oberkiefersituation und auf den gesamten 3D-Datensatz der Unterkiefersituation übertragen wird und
- die Konstruktion von Zahnoberflächen eines Zahnersatzteils sowie zur Herstellung dentaler Restaurationen unter Verwendung derart zugeordneter 3D-Datensätze erfolgt.

Vorteilhafterweise können nur Flächenpaare berücksichtigt werden, die aneinander vorbeigleiten können. Durch diese Auswahl werden Verfälschungen von Flächenpaaren, an denen kein Kontakt stattfinden kann, vermieden.

Vorteilhafterweise kann der Optimierungsalgorithmus eine durchdringungsfreie Gesamtsituation eines 3D-Datensatzes einer Oberkiefersituation und eines 3D-Datensatzes einer Unterkiefersituation bereitstellen. Durch diese Einschränkung bei der Optimierung kann das Arbeiten und Zuordnen eines realen Modells nachempfunden werden. Vorteilhafterweise kann eine näherungsweise Zuordnung des 3D-Datensatzes der Oberkiefersituation und des 3D-Datensatzes der Unterkiefersituation über eine Vorregistrierung erfolgen. Die Vorregistrierung hilft bei der Festlegung der sich zumindest näherungsweise gegenüberliegenden Flächenelementen.

Vorteilhafterweise können die ausgewählten Flächenelemente in Okklusalrichtung automatisch auf die jeweils andere Seite projiziert und damit ausgewählt werden. Damit ist eine automatische Übertragung von auf nur einer Seite ausgewählten Bereichen auf die andere möglich.

Vorteilhafterweise können gezielt nur die Flächenelemente ausgewählt werden, bei denen weiterer Optimierungsbedarf besteht.

Die Erfindung basiert also auf einem Verfahren, bei dem die Kontaktflächen näherungsweise markiert werden. Nicht markierte Flächen werden für die Kontakterzeugung nicht beachtet. Insgesamt wird aber auf eine durchdringungsfreie Gesamtsituation geachtet, wie sie bei echten Gipsmodellen im Artikulator auch gegeben ist. Es werden relevante und sich gegenüberliegende Flächenpaare identifiziert und per Optimierungsalgorithmus in lokale Abstandsminima unter Berücksichtigung von Flächen, die aneinander vorbeigleiten können gezogen, was die gewünschten Kontaktstellen ergibt.

Dabei kann eine Vorregistrierung der Kiefer erfolgen, um die Zuordnung der zueinander gerichteten Flächen zu vereinfachen, was anwenderfreundlich z.B. mit einer bukkalen Registrierung umsetzbar ist.

Die Erfindung betrifft weiterhin ein Computerprogramm, insbesondere ein CAD/CAM-Programm, welches mittels des erfindungsgemäßen Verfahrens steuerbar ist.

Vorzugsweise ist das Computerprogramm dazu eingerichtet, um mindestens einen 3D-Datensatz einer Oberkiefersituation oder einen 3D-Datensatz einer Unterkiefersituation auf einem Monitor darzustellen, wobei der 3D-Datensatz eine Markierung aufweist, die einen Zustand des Computerprogramms angibt, in welchen das Computerprogramm durch Zuordnung der 3D-Datensätze gebracht werden kann.

Die Erfindung betrifft weiter einen Datenträger, der das erfindungsgemäße Computerprogramm speichert.

Außerdem betrifft sie einen Rechner zur Steuerung eines Computerprogramms.

Kurze Beschreibung der Zeichnungen Ausführungsbeispiele der Erfindung sind in den Zeichnungen dargestellt und in der folgenden Beschreibung näher erläutert. Es zeigt:
- Fig. 1: einen 3D-Datensatz einer Oberkiefersituation und einen 3D-Datensatz der dazugehörigen Unterkiefersituation in einer Grobausrichtung zueinander
- Fig. 2: einen Schnitt durch die beiden zueinander grob ausgerichteten 3D-Datensätze als Prinzipskizze mit identifizierten gegenüberliegenden Flächenelementen,
- Fig. 3: die für eine Zuordnung in Frage kommenden ausgewählten Flächenelemente auf beiden Seiten,
- Fig. 4A: Bereiche, die durch Projektion in Okklusionsrichtung abgegrenzt werden;
- Fig. 4B: die abgegrenzten Bereiche aus Fig. 4A als gegenüberliegende markierte Flächenpaare;
- Fig. 4c: die für die Optimierung verwendeten ausgeschnittenen Bereiche aus Fig. 4B;
- Fig. 5: einen 3D-Datensatz des Oberkiefers nach einer Vorregistrierung mit einer Kontakt-Ausgangssituation,
- Fig. 6: den 3D-Datensatz aus Fig. 5 mit ausgewählten Flächenelementen,
- Fig. 7: den 3D-Datensatz des Oberkiefers aus Fig. 5 nach mit einer Kontaktsituation nach der Optimierung,
- Fig. 8: ein Vergleich des Oberkiefers als Gipsmodell mit einer Kontaktsituation im Artikulator bei maximaler Interkuspidation,
- Fig. 9: einen Datenträger, einen Rechner zur Durchführung des Verfahrens sowie eine Bearbeitungsmaschine zur Herstellung des Zahnersatzteils.

### Ausführungsbeispiel

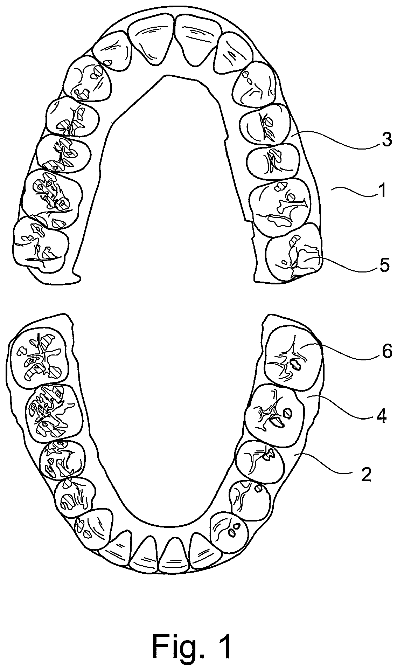

In einer Ausführungsform der Erfindung wird ein CAD/CAM-Programm auf einer Vorrichtung zur Konstruktion von Zahnoberflächen eines Zahnersatzteils und Herstellung von Zahnrestaurationen (beispielsweise CEREC AC + MC XL der Anmelderin) ausgeführt. Das CAD/CAM-Programm ist dazu eingerichtet, einen in Fig. 1 dargestellten 3D-Datensatz 1 einer Oberkiefersituation und ein 3D-Datensatz 2 einer Unterkiefersituation jeweils mit mehreren im jeweiligen Kieferbogen 3, 4 angeordneten Zähnen 5, 6 auf einem Bildschirm zur Anzeige zu bringen.

Aus dem 3D-Datensatz 1 der Oberkiefersituation und aus dem 3D-Datensatz 2 der Unterkiefersituation wird eine gemeinsame Okklusalrichtung in bekannter Weise automatisch bestimmt.

Weiterhin wird die Position der Zähne 5, 6 im Kieferbogen 3, 4 im 3D-Datensatz 1 der Oberkiefersituation und im 3D-Datensatz 2 der Unterkiefersituation in bekannter Weise automatisch bestimmt.

Da die Zähne 5 der Oberkiefersituation 1 und die Zähne 6 der Unterkiefersituation 2 bei Interkuspidation einen Vielpunktkontakt mit vielen Kontaktpunkten aufweisen, der bei getrennter Erfassung der 3D-Datensätze unbekannt ist, muss eine Zuordnung durch Korrelation der 3D-Datensätze erst hergestellt werden.

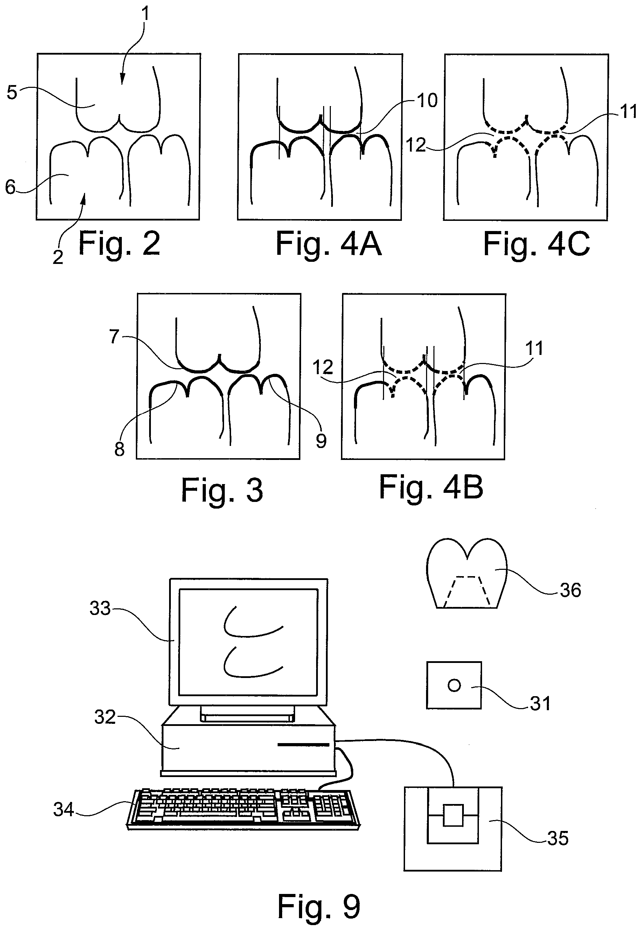

Die Vorgehensweise dazu ist wie folgt: anhand der Position der Zähne 5, 6 im Kieferbogen 3, 4 werden sich erwartungsgemäß zumindest näherungsweise gegenüberliegende Flächenelemente auf mindestens einer Seite, also auf einem der beiden Kiefersituationen automatisch identifiziert. In Fig. 2 ist ein Schnitt durch die beiden zueinander grob ausgerichteten 3D-Datensätze 1, 2 als Prinzipskizze mit an Zähnen 5, 6 identifizierten gegenüberliegenden Flächenelementen dargestellt.

Fig. 3 zeigt die für eine Zuordnung in Frage kommenden ausgewählten Flächenelemente 7-9 auf beiden Seiten, also in dem 3D-Datensatz 1 der Oberkiefersituation und in dem 3D-Datensatz 2 der Unterkiefersituation. Die Flächen, die in Kontakt treten sollen, werden automatisch oder vom Nutzer näherungsweise ausgewählt. Auf der anderen Seite der gegenüberliegenden Flächenelemente werden diejenigen Flächenelemente, die in Kontakt treten sollen, vom Nutzer und/oder automatisch näherungsweise ausgewählt.

Die Flächen können auf unterschiedliche Weise bestimmt werden: per Benutzereingabe, automatisch mithilfe der Zahngeometrie, insbesondere der Krümmung, automatisch mithilfe der Biogenerik, automatisch anhand eines Höhenparameters und man kann bestimmte Flächenanteile einschließen und bestimmte Flächenanteile ausschließen. Diese Auswahl kann vom Benutzer beispielsweise durch Anmalen des 3D Modells, Klicken, Umrandungslinien zeichnen vorgenommen werden und kann durch eine Vorregistrierung der Kiefer zueinander automatisch auf den Gegenkiefer übertragen werden.

Es können tendenziell dabei "zu viele" Flächen, also auch bereits welche mit bereits gutem Kontakt markiert werden oder gezielt nur die, bei denen weiterer Optimierungsbedarf besteht.

Fig. 4A zeigt Bereiche von zugeordneten Flächenelementen, die nach der Auswahl gemäß Fig. 3 durch eine automatisch ablaufende Projektion auf die jeweils andere Seite in Okklusionsrichtung, dargestellt durch die geraden Linien 10, abgegrenzt werden können. Fig. 4B zeigt die durch die Projektion abgegrenzten Bereiche aus Fig. 4A als gegenüberliegende ausgewählte Flächenpaare 11, 12 und Fig. 4c zeigt die für die Optimierung verwendeten ausgeschnittenen Bereiche aus Fig. 4B, die auf beiden Seiten der gegenüberliegenden Flächenelemente vorhanden sind und Flächenpaare 11, 12 bilden.

Die Kontaktoptimierung erfolgt dadurch, dass nur die ausgeschnittenen Flächenpaare mittels eines Optimierungsalgorithmus, z.B. ein Downhill-Simplex mit geeigneter Bewertungsfunktion der Flächen, im Abstand zueinander minimiert werden. Die Kontaktsituation wird dadurch verbessert, dass nicht das gesamte Kiefermodell, sondern nur diese nicht zusammenhängenden Bereiche verwendet werden. Die optimierte Zuordnung der Flächenpaare wird auf den gesamten 3D-Datensatz der Oberkiefersituation und auf den gesamten 3D-Datensatz der Unterkiefersituation übertragen.

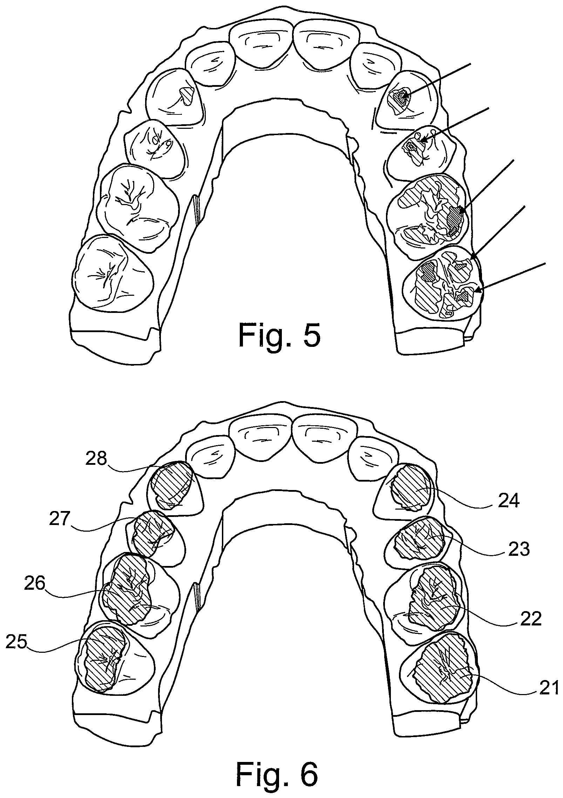

Die Fig. 5-8 zeigen einen Screenshot von den eingezeichneten Flächen und die vorher/nachher Situation verglichen mit der tatsächlichen echten Kontaktsituation am Gipsmodell im Artikulator. Dabei ist zu erkennen, dass es sogar ebenfalls möglich ist, vorhandene Durchdringungen einer Vorregistrierung aufzulösen, sowie die gewünschten echten Kontakte zu erzeugen. Außerdem wird im Vergleich mit Modell im Artikulator mit per Kontaktfolie gefärbten echten Kontakten gezeigt, wie realistisch und exakt das errechnete Ergebnis ist.

Fig. 5 zeigt einen 3D-Datensatz des Oberkiefers nach einer Vorregistrierung mit einer Kontakt-Ausgangssituation. Im Beispiel hat die eine Kieferhälfte zu wenig, die andere Kieferhälfte tendenziell zu starke Kontakte, angedeutet durch die Pfeile und sogar Durchdringungen

Zur Ausführung des erfindungsgemäßen Verfahrens werden wie in Fig. 6 gezeigt in dem 3D-Datensatzes aus Fig. 5 Flächenelemente 21-28 an den Zähnen ausgewählt im Beispiel auf beide Kieferseiten, da auf beiden Seiten Optimierungsbedarf der Vorregistrierung besteht.

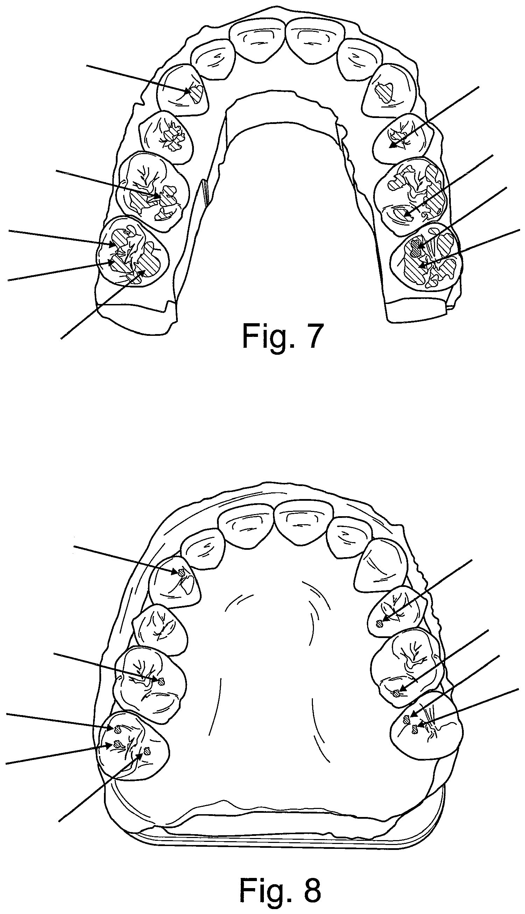

In Fig. 7 Ist der 3D-Datensatz des Oberkiefers aus Fig. 5 mit einer Kontaktsituation nach der Optimierung dargestellt, wobei die Kontaktpunkte wieder durch Pfeile angedeutet sind. Ein Vergleich mit der in Fig. 8 dargestellten Kontaktsituation eines Oberkiefers als Gipsmodell im Artikulator bei maximaler Interkuspidation zeigt eine Übereinstimmung an allen den wesentlichen Kontaktpunkten, die auch hier durch Pfeile angegeben sind.

Anhand eines derartig zugeordneten Modells kann die Konstruktion von Zahnoberflächen eines Zahnersatzteils sowie zur Herstellung dentaler Restaurationen unter Verwendung derart zugeordneter 3D-Datensätze erfolgen.

Ein sequentielles Vorgehen, z.B. zum Erzeugen einer Bisserhöhung, ist denkbar, so dass z.B. auch nach einer erfolgten Restaurationserzeugung das Verfahren erneut auf Modell-Scan inklusive dann berechneter Restaurationen angewendet werden kann.

Fig. 9 zeigt einen Datenträger 31, auf dem das Computerprogramm gespeichert ist, einen Rechner 32 mit Monitor 33 und Eingabemittel 34 zur Durchführung des Verfahrens sowie eine Bearbeitungsmaschine 35 zur Herstellung eines Zahnersatzteils 36, das als Krone in starker Vergrößerung dargestellt ist.

## Patentansprüche

1. Verfahren zur Konstruktion von Zahnoberflächen eines Zahnersatzteils sowie zur Herstellung dentaler Restaurationen, ausgehend von einem 3D-Datensatz einer Oberkiefersituation (1) und einem 3D-Datensatz einer Unterkiefersituation (2) jeweils mit mehreren im jeweiligen Kieferbogen angeordneten Zähnen (5, 6), wobei die Zähne der Oberkiefersituation und die Zähne der Unterkiefersituation bei Interkuspidation einen Vielpunktkontakt mit vielen Kontaktpunkten aufweisen, wobei die Zuordnung mit einer Kontaktoptimierung erfolgt, indem
- aus dem 3D-Datensatz der Oberkiefersituation und aus dem 3D-Datensatz der Unterkiefersituation eine gemeinsame Okklusalrichtung automatisch bestimmt wird,
- wobei die Position der Zähne im Kieferbogen im 3D-Datensatz der Oberkiefersituation und im 3D-Datensatz der Unterkiefersituation automatisch bestimmt wird,
- wobei anhand der Position der Zähne im Kieferbogen sich erwartungsgemäß zumindest näherungsweise gegenüberliegende Flächenelemente (7, 8, 9, 21-28) auf mindestens einer Seite automatisch identifiziert werden und
- wobei auf mindestens einer Seite der gegenüberliegenden Flächenelemente diejenigen Flächenelemente, die in Kontakt treten sollen, vom Nutzer und/oder automatisch näherungsweise ausgewählt werden;
- wobei auf der anderen Seite der gegenüberliegenden Flächenelemente diejenigen Flächenelemente, die in Kontakt treten sollen, vom Nutzer und/oder automatisch näherungsweise ausgewählt werden;
- wobei Bereiche, die auf beiden Seiten der gegenüberliegenden Flächenelemente vorhanden sind, ausgeschnitten werden und Flächenpaare (11, 12) bilden,
- wobei nur die ausgeschnittenen Flächenpaare mittels eines Optimierungsalgorithmus im Abstand zueinander minimiert werden, wobei
- die optimierte Zuordnung der Flächenpaare auf den gesamten 3D-Datensatz der Oberkiefersituation und auf den gesamten 3D-Datensatz der Unterkiefersituation übertragen wird und
- wobei die Konstruktion von Zahnoberflächen eines Zahnersatzteils sowie zur Herstellung dentaler Restaurationen unter Verwendung derart zugeordneter 3D-Datensätze erfolgt.

2. Verfahren nach Anspruch 1, **dadurch gekennzeichnet, dass** nur Flächenpaare berücksichtigt werden, die aneinander vorbeigleiten können.

3. Verfahren nach Anspruch 1 oder 2, **dadurch gekennzeichnet, dass** der Optimierungsalgorithmus eine durchdringungsfreie Gesamtsituation eines 3D-Datensatzes einer Oberkiefersituation und eines 3D-Datensatzes einer Unterkiefersituation bereitstellt.

4. Verfahren nach einem der Ansprüche 1 bis 3, **dadurch gekennzeichnet, dass** eine näherungsweise Zuordnung des 3D-Datensatzes der Oberkiefersituation und des 3D-Datensatzes der Unterkiefersituation über eine Vorregistrierung erfolgt, insbesondere über eine bukkale Registrierung.

5. Verfahren nach einem der Ansprüche 1 bis 4, **dadurch gekennzeichnet, dass** die ausgewählten Flächenelemente in Okklusalrichtung automatisch auf die jeweils andere Seite projiziert werden und dass die Bereiche, die auf beiden Seiten der gegenüberliegenden Flächenelemente vorhanden sind, ausgeschnitten werden und Flächenpaare bilden.

6. Verfahren nach einem der Ansprüche 1 bis 5, **dadurch gekennzeichnet, dass** gezielt nur die Flächenelemente ausgewählt werden, bei denen weiterer Optimierungsbedarf besteht.

7. Computerprogramm, **dadurch gekennzeichnet, dass** es ein Verfahren nach einem der Ansprüche 1 bis 6 implementiert.

8. Computerprogramm nach Anspruch 7, **dadurch gekennzeichnet, dass** es ein CAD/CAM-Programm ist.

9. Computerprogramm nach Anspruch 7 oder 8, **dadurch gekennzeichnet, dass** es eingerichtet ist, um mindestens einen 3D-Datensatz einer Oberkiefersituation oder einen 3D-Datensatz einer Unterkiefersituation auf einem Monitor (41) darzustellen, wobei der 3D-Datensatz eine Markierung aufweist, die einen Zustand des Computerprogramms angibt, in welchen das Computerprogramm durch Zuordnung der 3D-Datensätze gebracht werden kann.

## Claims

1. Method for constructing tooth surfaces of a dental prosthesis, as well as for producing dental restorations, based upon a 3-D data set of a maxilla situation (1) and a 3-D data set of a mandible situation (2), each with multiple teeth (5, 6) arranged in the respective jaw arch, wherein, with intercuspation, the teeth of the maxilla situation and the teeth of the mandible situation exhibit a multipoint contact with numerous contact points, wherein the association is effected by a contact optimization, in that
- a common occlusal direction is automatically determined from the 3-D data set of the maxilla situation and the 3-D data set of the mandible situation,
- wherein the position of the teeth in the jaw arch is automatically determined in the 3-D data set of the maxilla situation and in the 3-D data set of the mandible situation,
- wherein at least approximately opposing surface elements (7, 8, 9, 21-28) on at least one side are automatically identified as expected using the position of the teeth in the jaw arch, and
- wherein those surface elements that are to come into contact are approximately selected by the user and/or automatically on at least one side of the opposing surface elements;
- wherein those surface elements that are to come into contact are approximately selected by the user and/or automatically on the other side of the opposing surface elements;
- wherein regions that are present on both sides of the opposing surface elements are excised and form surface pairs (11, 12),
- wherein only the excised surface pairs are minimized, by means of an optimization algorithm, at a distance from one another, wherein
- the optimized association of the surface pairs is transferred to the complete 3-D data set of the maxilla situation and to the complete 3-D data set of the mandible situation, and
- wherein the construction of tooth surfaces of a dental prosthesis, as well as for production of dental restorations, is effected using such associated 3-D data sets.

2. Method according to claim 1, **characterized in that** only surface pairs that are capable of sliding past one another are considered.

3. Method according to claim 1 or 2, **characterized in that** the optimization algorithm provides an intersection-free, overall situation of a 3-D data set of a maxilla situation and of a 3-D data set of a mandible situation.

4. Method according to one of claims 1 through 3, **characterized in that** an approximate association of the 3-D data set of the maxilla situation and of the 3-D data set of the mandible situation is effected via a pre-registration - especially, via a buccal registration.

5. Method according to one of claims 1 through 4, **characterized in that** the selected surface elements are automatically projected onto the respective other side in the occlusal direction, and **in that** the regions that are present on both sides of the opposing surface elements are excised and form surface pairs.

6. Method according to one of claims 1 through 5, **characterized in that** specifically only the surface elements in which there exists a further need for optimization are selected.

7. Computer program, **characterized in that** it implements a method according to one of claims 1 through 6.

8. Computer program according to claim 7, **characterized in that** it is a CAD/CAM program.

9. Computer program according to claim 7 or 8, **characterized in that** it is set up to present at least one 3-D data set of a maxilla situation or a 3-D data set of a mandible situation on a monitor (41), wherein the 3-D data set has a marking that indicates a state of the computer program into which the computer program may be brought via association of the 3-D data sets.

## Revendications

1. Procédé pour la construction de surfaces dentaires d'une prothèse dentaire ainsi que pour la réalisation de restaurations dentaires, partant d'un ensemble de données 3D d'une situation maxillaire (1) et d'un ensemble de données 3D d'une situation mandibulaire (2) présentant à chaque fois plusieurs dents (5, 6) disposées dans l'arcade de la mâchoire respective, les dents de la situation maxillaire et les dents de la situation mandibulaire présentant un contact à points multiples, présentant de nombreux points de contact, lors de l'intercuspidation, l'attribution ayant lieu avec une optimisation de contact, en ce que
- on détermine automatiquement une direction occlusale commune à partir de l'ensemble de données 3D de la situation maxillaire et à partir de l'ensemble de données 3D de la situation mandibulaire,
- la position des dents dans l'arcade de la mâchoire dans l'ensemble de données 3D de la situation maxillaire et dans l'ensemble de données 3D de la situation mandibulaire étant déterminée automatiquement,
- des éléments de surface (7, 8, 9, 21-28) dont on s'attend à ce qu'ils se trouvent au moins approximativement les uns en face des autres sur au moins un côté étant identifiés automatiquement à l'aide de la position des dents dans l'arcade de la mâchoire et
- les éléments de surface qui doivent entrer en contact sur au moins un côté des éléments de surface, qui se trouvent les uns en face des autres, étant choisis approximativement par l'utilisateur et/ou automatiquement ;
- les éléments de surface qui doivent entrer en contact sur l'autre côté des éléments de surface, qui se trouvent les uns en face des autres, étant choisis approximativement par l'utilisateur et/ou automatiquement ;
- les zones, qui se trouvent sur les deux côtés des éléments de surface, se trouvant les uns en face des autres, étant découpées et formant des paires de surfaces (11, 12),
- seules les paires de surfaces découpées étant minimisées, en ce qui concerne leur distance l'une par rapport à l'autre, par un algorithme d'optimisation,
- l'attribution optimisée des paires de surfaces étant transférée à tout l'ensemble de données 3D de la situation maxillaire et à tout l'ensemble de données 3D de la situation mandibulaire et
- la construction des surfaces dentaires d'une prothèse dentaire ainsi que la réalisation de restaurations dentaires ayant lieu avec utilisation des ensembles de données 3D ainsi attribués.

2. Procédé selon la revendication 1, **caractérisé en ce que** seules des paires de surfaces qui peuvent glisser les unes sur les autres sont prises en considération.

3. Procédé selon la revendication 1 ou 2, **caractérisé en ce que** l'algorithme d'optimisation met à disposition une situation globale sans pénétration d'un ensemble de données 3D d'une situation maxillaire et d'un ensemble de données 3D d'une situation mandibulaire.

4. Procédé selon l'une quelconque des revendications 1 à 3, **caractérisé en ce qu'**une attribution approximative de l'ensemble de données 3D de la situation maxillaire et de l'ensemble de données 3D de la situation mandibulaire a lieu via un pré-enregistrement, en particulier via un enregistrement buccal.

5. Procédé selon l'une quelconque des revendications 1 à 4, **caractérisé en ce que** les éléments de surface choisis sont projetés dans la direction occlusale automatiquement sur l'autre côté respectif et **en ce que** les zones qui sont présentes sur les deux côtés des éléments de surface se trouvant l'un en face de l'autre sont découpées et forment les paires de surfaces.

6. Procédé selon l'une quelconque des revendications 1 à 5, **caractérisé en ce que** seuls les éléments de surface pour lesquels il existe un autre besoin d'optimisation sont choisis de manière ciblée.

7. Programme informatique, **caractérisé en ce qu'**il met en oeuvre un procédé selon l'une quelconque des revendications 1 à 6.

8. Programme informatique selon la revendication 7, **caractérisé en ce qu'**il s'agit d'un programme de CAO/FAO.

9. Programme informatique selon la revendication 7 ou 8, **caractérisé en ce qu'**il est conçu pour représenter au moins un ensemble de données 3D d'une situation maxillaire ou un ensemble de données 3D d'une situation mandibulaire sur un écran (41), l'ensemble de données 3D présentant un repère qui indique un état du programme informatique dans lequel le programme informatique peut être amené par attribution des ensembles de données 3D.
